# DEMANDE DE BREVET EUROPEEN

(11) **EP 1 927 336 A2**
(43) Date de publication de la demande: **04.06.2008**
(21) Numéro de dépôt: 08152449.8
(22) Date de dépôt: 18.06.2001
(51) Int. Cl.: A61K 8/06, A61K 8/37, A61K 8/72, A61K 8/81, A61Q 19/00

(54) **Nouveaux latex inverses autoinversibles sur des esters d'acides gras, compositions cosmétiques, dermocosmétiques, dermopharmaceutiques ou pharmaceutiques en comportant**

(30) Priorité: 28.06.2000 FR 0008303
(62) Demande divisionnaire de: 01401595.2
(71) Demandeur: SOCIETE D'EXPLOITATION DE PRODUITS POUR LES INDUSTRIES CHIMIQUES, S.E.P.P.I.C., 75007 Paris (FR)
(72) Inventeur: Tabacchi, Guy, 75012 Paris (FR); Boiteux, Jean-Pierre, 81710, Saix (FR); Amalric, Chantal, 81700 Blan (FR); Michel, Nelly, 94700 Maisons Alfort (FR); Mallo, Paul, 78290 Croissy-sur-Seine (FR)
(74) Mandataire: Conan, Philippe Claude

(57) **Abrégé**

Composition comprenant une phase huile, une phase aqueuse, au moins un agent émulsifiant de type eau dans huile (E/H), au moins un agent émulsifiant de type huile dans eau (H/E), sous forme d'un latex inverse auto-inversible comprenant de 20% à 70% en poids d'un polyélectrolyte branché ou réticulé caractérisé en ce que la phase huile est essentiellement constituée d'un composé de formule (I) :

R₁-(C=O)-O-[[CH₂-CH[O-[C(=O)]ₘ-R₂]-CH₂-O]ₙ-[C(=O)]ₚ]_{q}-R₃ (I)

dans laquelle :
R₁ représente une chaîne hydrocarbonée,
R₂ représente indépendamment de R₁, un atome d'hydrogène ou une chaîne hydrocarbonée
R₃ représente indépendamment de R₁ ou de R₂, un atome d'hydrogène ou une chaîne hydrocarbonée, m, n et p sont indépendamment l'un de l'autre, égaux à 0 ou à 1, et q est égal à 1. Composition cosmétique, dermocosmétique, dermopharmaceutique ou pharmaceutique en comportant.

## Description

La présente demande de brevet concerne des latex inverses eau dans huile, leur procédé de préparation et leur application en tant qu'épaississants et/ou émulsionnants pour des produits de soins de la peau et des cheveux ou pour la fabrication de préparations cosmétiques, dermocosmétiques, dermopharmaceutiques ou pharmaceutiques.

Des polymères épaississants synthétiques, se présentant sous forme de latex inverses, sont décrits comme pouvant être utilisés dans la fabrication de compositions topiques, dans les demandes de brevet français publiées sous les numéros 2721511, 2733805, 2774688, 2774996 et 2782086 ainsi que dans la demande de brevet européen publiée sous le numéro EP 0 503 853.

Les demande internationales WO 99/36445 A1 et WO 99/42521 A1 ainsi que la demande de brevet européen publiée sous le numéro EP 1 055 707 A1 et citée au titre de l'article 54(3) CBE, divulguent des latex inverses à base d'acide 2-méthyl 2-[(1-oxo 2-propényl) amino] 1-propanesulfonique partiellement ou totalement salifié ainsi que des composition cosmétiques dans lesquelles la phase grasse est un ester d'acide gras.

Cependant, certains d'entre eux engendrent parfois des réactions d'intolérance de certaines peaux sensibles.

C'est pourquoi la demanderesse s'est intéressée à la recherche de nouvelles émulsions de polymères, qui soient mieux tolérées par la peau que celles de l'état de la technique.

L'invention a pour objet une composition comprenant une phase huile, une phase aqueuse, au moins un agent émulsifiant de type eau dans huile (E/H), au moins un agent émulsifiant de type huile dans eau (H/E), sous forme d'un latex inverse auto-inversible comprenant de 20% à 70% en poids, et de préférence de 25% à 50% en poids, d'un polyélectrolyte branché ou réticulé, choisi parmi :
- un homopolymère de l'acide 2-méthyl 2-[(1-oxo 2-propènyl) amino] 1-propanesulfonique partiellement ou totalement salifiée sous forme d'un sel de métal alcalin, de sel d'ammonium, de sel de monoéthanolamine ou de sel de lysine ;
- un homopolymère de l'acide acrylique, de l'acide méthacrylique, de l'acide itaconique ou de l'acide maléique partiellement ou totalement salifié ;
- un copolymère de l'acide 2-méthyl 2-[(1-oxo 2-propènyl) amino] 1-propanesulfonique partiellement ou totalement salifiée sous forme d'un sel de métal alcalin, de sel d'ammonium, de sel de monoéthanolamine ou de sel de lysine, copolymérisé ou bien avec de l'acide acrylique, de l'acide méthacrylique, de l'acide itaconique ou de l'acide maléique partiellement ou totalement salifié , ou bien avec au moins un monomère neutre choisi parmi l'acrylate de (2-hydroxy éthyle), l'acrylate de (2,3-dihydroxy propyle), le méthacrylate de (2-hydroxy éthyle), le méthacrylate de (2,3-dihydroxy propyle), ou un dérivé éthoxylé de poids moléculaire compris entre 400 et 1000, de chacun de ces esters ;
- un copolymère de l'acide acrylique, de l'acide méthacrylique, de l'acide itaconique ou de l'acide maléique partiellement ou totalement salifié, copolymérisé avec au moins un monomère neutre choisi parmi l'acrylate de (2-hydroxy éthyle), l'acrylate de (2,3-dihydroxy propyle), le méthacrylate de (2-hydroxy éthyle), le méthacrylate de (2,3-dihydroxy propyle), ou un dérivé éthoxylé de poids moléculaire compris entre 400 et 1000, de chacun de ces esters ; caractérisé en ce que la phase huile est essentiellement constituée d'un composé de formule (I) :

   R₁-(C=O)-O-[[CH₂-CH[O-[C(=O)]ₘ-R₂]-CH₂-O]ₙ-[C(=O)]ₚ]_{q}-R₃ (I)
Dans laquelle :
R₁ représente une chaîne hydrocarbonée saturée ou insaturée, linéaire ou ramifiée comportant de 7 à 30 atomes de carbone,
R₂ représente indépendamment de R₁, un atome d'hydrogène, une chaîne hydrocarbonée saturée ou insaturée linéaire ou ramifiée comportant de 7 à 30 atomes de carbone,
R₃ représente indépendamment de R₁ ou de R₂, un atome d'hydrogène ou une chaîne hydrocarbonée saturée ou insaturée, linéaire ou ramifiée comportant de 1 à 30 atomes de carbone,
m, n et p sont indépendamment l'un de l'autre, égaux à 0 ou à 1, et q est égal à 1.

Dans la formule (I) telle que définie précédemment, R₁, R₂ et R₃ représentent notamment, indépendamment les uns des autres, un radical choisi parmi les radicaux heptyle, octyle, nonyle, décyle, undécyle, dodécyle, tridécyle, tétradécyle, pentadécyle, hexadécyle, heptadécyle, octadécyle, nonadécyle, eicosyle, heneicosyle, docosyle, heptadécènyle, eicosènyle, heneicosènyle, docosènyle, heptadécadiènyle ou décènyle ; le groupe R₁-C(=O)- représente plus particulièrement l'un des radicaux octanoyle (caprylyle), décanoyle, undécylènoyle, dodécanoyle (lauroyle), tétradécanoyle (myristyle), hexadécanoyle (palmitoyle), octadécanoyle (stéaryle), eicosanoyle (arachidoyle), docosanoyle (béhènoyle), 8-octadécènoyle (oléyle), éicosènoyle (gadoleoyle), 13-docosènoyle (érucyle), 9,12-octadécadiènoyle (linoléoyle) ou 9,12,15-octadécatriénoyle (linolénoyle).

Selon un premier aspect particulier de la présente invention, la phase huile du latex inverse, est essentiellement constituée d'un composé de formule (Ia) :

R₁-(C=O)-O-CH₂-CH[O-[C(=O)]ₘ-R₂]-CH₂-O-[C(=O)]ₚ-R₃ (Ia)

Correspondant à la formule (I) telle définie précédemment dans laquelle q et n sont égaux à 1, ou un mélange de composés de formule (Ia).

Lorsque la phase huile du latex inverse, est essentiellement constituée d'un composé de formule (Ia), il s'agit de préférence,
ou bien d'un composé de formule (Ia₁) :

R₁-(C=O)-O-CH₂-CH(OH)-CH₂-OH (Ia₁)

correspondant à la formule (Ia) telle définie précédemment, dans laquelle m et p sont égaux à 0 et R₂ et R₃ représentent un atome d'hydrogène,
ou bien d'un composé de formule (Ia₂) :

R₁-(C=O)-O-CH₂-CH(OH)-CH₂-O-C(=O)-R₃ ) (Ia₂

correspondant à la formule (Ia) telle définie précédemment dans laquelle p est égal à 1, m est égal à 0 et R₂ représente un atome d'hydrogène,
ou bien d'un composé de formule (Ia₃) :

R₁-(C=O)-O-CH₂-CH[O-C(=O)-R₂]-CH₂-O-C(=O)-R₃ (Ia₃)

correspondant à la formule (Ia) telle définie précédemment dans laquelle m et p sont égaux à 1,
ou bien d'un mélange de composés de formules (Ia₁), (Ia₂) et/ou (Ia₃).

Comme exemples de composés de formules (Ia₁), (Ia₂) ou (Ia₃), il y a par exemple les triglycérides d'acides gras ou de mélanges d'acides gras tels que le mélange de triglycérides d'acides gras comportant de 6 à 10 atomes de carbone, commercialisé sous le nom SOFTENOL^{™} 3819, le mélange de triglycérides d'acides gras comportant de 8 à 10 atomes de carbone, commercialisé sous le nom SOFTENOL^{™} 3108, le mélange de triglycérides d'acides gras comportant de 8 à 18 atomes de carbone, commercialisé sous le nom SOFTENOL^{™} 3178, le mélange de triglycérides d'acides gras comportant de 12 à 18 atomes de carbone, commercialisé sous le nom SOFTENOL^{™} 3100, le mélange de triglycérides d'acides gras comportant 7 atomes de carbone commercialisé sous le nom SOFTENOL^{™} 3107, le mélange de triglycérides d'acides gras comportant 14 atomes de carbone commercialisé sous le nom SOFTENOL^{™} 3114 ou le mélange de triglycérides d'acides gras comportant 18 atomes de carbone commercialisé sous le nom SOFTENOL^{™} 3118, le dilaurate de glycérol, le dioléate de glycérol, l'isostéarate de glycérol, le distéarate de glycérol, le monolaurate de glycérol, le monooléate de glycérol, le monoisostéarate de glycérol, le monostéarate de glycérol, ou un mélange de ces composés.

Par polymère branché, on désigne un polymère non linéaire qui possède des chaînes pendantes de manière à obtenir, lorsque ce polymère est mis en solution dans l'eau, un fort état d'enchevêtrement conduisant à des viscosités à bas gradient très importantes.

Par polymère réticulé, on désigne un polymère non linéaire se présentant à l'état de réseau tridimensionnel insoluble dans l'eau, mais gonflable à l'eau et conduisant donc à l'obtention d'un gel chimique.

La composition selon l'invention peut comporter des motifs réticulés et/ou des motifs branchés.

Par "agent émulsifiant du type eau dans huile", on désigne des agents émulsifiants possédant une valeur de HLB suffisamment faible pour fournir des émulsions eau dans huile tels que les polymères tensioactifs commercialisés sous le nom de HYPERMER^{™} ou tels que les esters de sorbitan, comme le monooléate de sorbitan commercialisé par la Société SEPPIC sous le nom de marque MONTANE^{™} 80 ou l'isostéarate de sorbitan commercialisé par SEPPIC sous le nom de MONTANE^{™} 70. On peut aussi adjoindre à ces agents émulsifiants, l'oléate de sorbitan éthoxylé avec 5 moles d'oxyde d'éthylène, commercialisé par la société SEPPIC sous le nom de MONTANOX^{™} 81.

Par "agent émulsifiant du type huile dans eau", on désigne des agents émulsifiants possédant une valeur de HLB suffisamment élevée pour fournir des émulsions huile dans l'eau tels que les esters de sorbitan éthoxylés comme l'oléate de sorbitan éthoxylé avec 20 moles d'oxyde d'éthylène, commercialisé par la société SEPPIC sous le nom de MONTANOX^{™} 80, l'huile de ricin éthoxylée à 40 moles d'oxyde d'éthylène commercialisée par la société SEPPIC sous le nom de SIMULSOL^{™} OL50, le laurate de sorbitan éthoxylé à 20 moles d'oxyde d'éthylène commercialisé par la société SEPPIC sous le nom de MONTANOX^{™} 20 ou l'alcool laurique éthoxylé à 7 moles d'oxyde d'éthylène, commercialisé par la société SEPPIC sou le nom de SIMULSOL^{™} P7.

Comme agents émulsifiants possédant une valeur de HLB suffisamment élevée pour fournir des émulsions huile dans l'eau, il y a aussi les composés de formule (II) :

R₄-O-[CH(R₅)-CH₂-O]ₙ-(G)ₓ-H (II)

dans laquelle R₄ représente un radical hydrocarboné linéaire ou ramifié, saturé ou insaturé comprenant de 1 à 30 atomes de carbones, R₅ représente un atome d'hydrogène ou radical alkyle comprenant 1 ou 2 atomes de carbone, G représente le reste d'un saccharide, x représente un nombre décimal compris entre 1 et 5, et n est égal, soit à zéro, soit à un nombre entier compris entre 1 et 30.

Par reste d'un saccharide, on désigne pour G, un radical bivalent résultant de l'enlèvement sur une molécule de sucre, d'une part d'un atome d'hydrogène d'un groupe hydroxyle et d'autre part du groupe hydroxyle anomérique. Le terme saccharide désigne notamment le glucose ou dextrose, le fructose, le mannose, le galactose, l'altrose, l'idose, l'arabinose, le xylose, le ribose, le gulose, le lyxose, le maltose, le maltotriose, le lactose, le cellobiose, le dextrane, le talose, l'allose, le raffinose, le lévoglucane, la cellulose ou l'amidon. La structure oligomérique (G)ₓ peut se présenter sous toute forme d'isomérie, qu'il s'agisse d'isomérie optique, d'isomérie géométrique ou d'isomérie de position ; elle peut aussi représenter un mélange d'isomères. Dans la formule (II) telle que définie précédemment, le radical R₄-O-[CH(R₅)-CH₂-O]ₙ- est lié à G, par le carbone anomérique de manière à former une fonction acétal. Le groupe divalent -[CH(R₅)-CH₂-O]ₙ- représente, soit une chaîne composée uniquement de groupes éthoxyle (R₅ = H), soit une chaîne composée uniquement de groupes propoxyle (R₅ = CH₃), soit une chaîne composée à la fois de groupes éthoxyle et de groupes propoxyle. Dans ce dernier cas, les fragments -CH₂-CH₂-O- et -CH(CH₃)-CH₂-O- sont distribués dans ladite chaîne, de façon séquencée ou aléatoire. Le nombre x, qui représente dans la formule (II) le degré moyen de polymérisation du saccharide, est plus particulièrement compris entre 1 et 3, notamment entre 1,05 et 2,5, tout particulièrement entre 1,1 et 2,0 et de préférence, inférieur ou égal à 1,5. Comme agents tensioactifs émulsifiants possédant une valeur de HLB suffisamment élevée pour fournir des émulsions huile dans l'eau, il y a plus particulièrement, les composés de formule (II) telle que définie précédemment dans laquelle G représente le reste du glucose ou le reste du xylose et/ou dans laquelle n est égal à 0, et/ou dans laquelle R₄ représente un radical comportant de 8 à 18 atomes de carbone et tout particulièrement dans laquelle R₄ représente plus particulièrement un radical octyle, décyle, undécyle, dodécyle, tétradécyle ou hexadécyle, lesdits radicaux étant linéaires ou ramifiés.

Comme exemples de produits commerciaux contenant lesdits composés, il y a par exemple :
Le SIMULSOL^{™} SL8, commercialisé par la société SEPPIC, qui est une solution aqueuse contenant entre environ 35% et 45% en poids d'un mélange d'alkyl polyglycosides consistant en entre 45% en poids et 55% en poids d'un composé de formule (II), dans laquelle G représente le reste du glucose, x est égal à environ 1,45, n est égal à 0 et R₄ représente un radical décyle, et entre 45% en poids et 55% en poids d'un composé de formule (II), dans laquelle G représente le reste du glucose, x est égal à environ 1,45, n est égal à 0 et R₄ représente un radical octyle ;
Le SIMULSOL^{™} SL10, commercialisé par la société SEPPIC, qui est une solution aqueuse contenant entre environ 40% en poids et 50% en poids d'un mélange d'alkyl polyglycosides, consistant en environ 85% en poids d'un composé de formule (II), dans laquelle G représente le reste du glucose, x est égal à environ 1,45, n est égal à 0 et R₄ représente un radical décyle, environ 7,5% en poids d'un composé de formule (II), dans laquelle G représente le reste du glucose, x est égal à environ 1,45, n est égal à 0 et R₄ représente un radical dodécyle et environ 7,5% en poids d'un composé de formule (II), dans laquelle G représente le reste du glucose, x est égal à environ 1,45, n est égal à 0 et R₄ représente un radical tétradécyle ;
Le SIMULSOL^{™} SL11, commercialisé par la société SEPPIC, qui est une solution aqueuse contenant entre environ 40% en poids et 50% en poids d'un mélange d'alkyl polyglycosides de formule (II), dans laquelle G représente le reste du glucose, x est égal à environ 1,45, n est égal à 0 et R₄ représente un radical undécyle ; ou
Le SIMULSOL^{™} SL26, commercialisé par la société SEPPIC, qui est une solution aqueuse contenant entre environ 40% en poids et 55% en poids d'un mélange d'alkyl polyglycosides consistant en environ 70% en poids d'un composé de formule (II), dans laquelle G représente le reste du glucose, x est égal à environ 1,45, n est égal à 0 et R₄ représente un radical dodécyle, environ 25% en poids d'un composé de formule (II), dans laquelle G représente le reste du glucose, x est égal à environ 1,45, n est égal à 0 et R₄ représente un radical tétradécyle et environ 5% en poids d'un composé de formule (II), dans laquelle G représente le reste du glucose, x est égal à environ 1,45, n est égal à 0 et R₄ représente un radical hexadécyle.

Selon un deuxième aspect particulier de la présente invention, le polyélectrolyte compris dans le latex inverse tel que défini précédemment, est un homopolymère de l'acide acrylique partiellement ou totalement salifié sous forme de sel de sodium ou de sel d'ammonium.

Selon un troisième aspect particulier de la présente invention, le polyélectrolyte compris dans le latex inverse tel que défini précédemment, est un copolymère d'acide 2-méthyl 2-[(1-oxo 2-propènyl) amino] 1-propanesulfonique partiellement ou totalement salifié (a), et d'acrylate de (2-hydroxy éthyle) (b), dans un rapport molaire (a) / (b) compris entre 30 / 70 et 90 / 10 et tout particulièrement 50 / 50 et 90 / 10. Le polyélectrolyte est de préférence un copolymère de sel de sodium ou de sel d'ammonium de l'acide 2-méthyl 2-[(1-oxo 2-propènyl) amino] 1-propanesulfonique (a₁) et de 10% à 40% d'acrylate de (2-hydroxy éthyle) (b), dans un rapport molaire (a₁) / (b), compris entre 60 / 40 et 90 / 10.

Selon un quatrième aspect particulier de la présente invention, le polyélectrolyte compris dans le latex inverse tel que défini précédemment, est un copolymère de sel de sodium, de sel d'ammonium, de sel de monoéthanolamine ou de sel de lysine de l'acide 2-méthyl 2-[(1-oxo 2-propènyl) amino] 1-propanesulfonique (a₁) et d'acide acrylique partiellement ou totalement salifié sous forme de sel de sodium, de sel d'ammonium, de sel de monoéthanolamine ou de sel de lysine (c₁), dans un rapport molaire (a₁) / (c₁), compris entre 30 / 70 et 90/10 et tout particulièrement entre 30 / 70 et 45 / 55.

Selon un cinquième aspect particulier de la présente invention, le polyélectrolyte compris dans le latex inverse tel que défini précédemment, est un copolymère de sel de sodium ou de sel d'ammonium de l'acide 2-méthyl 2-[(1-oxo 2-propènyl) amino] 1-propanesulfonique (a₂) et d'acrylamide (d), dans un rapport molaire (a₂) / (d), compris entre 50 / 50 et 30/70.

L'invention a plus particulièrement pour objet une composition telle que définie précédemment, caractérisée en ce que le polyélectrolyte est réticulé et/ou branché avec un composé diéthylénique ou polyéthylénique dans la proportion molaire exprimée par rapport aux monomères mis en oeuvre, de 0,005% à 1%, plus particulièrement de 0,01 % à 0,5 % et tout particulièrement de 0,1 % à 0,25 %. L'agent de réticulation et/ou l'agent de ramification est choisi parmi l'acide diallyoxyacétique ou un de ses sels comme le diallyloxyacétate de sodium, le diméthacrylate d'éthylèneglycol, le diacrylate d'éthylèneglycol, le diallyl urée, le triméthylol propanetriacrylate, le méthylène-bis(acrylamide), le triallylamine ou un mélange de ces composés.

Le latex inverse tel que défini ci-dessus, contient généralement de 4% à 10% en poids, d'agents émulsifiants. Généralement de 20% à 50% et plus particulièrement de 25% à 40% du poids total des émulsifiants sont du type eau dans huile et de 80% à 50% et plus particulièrement de 75 à 60% sont du type huile dans eau.

Sa phase huile représente de 15% à 40%, et de préférence de 20% à 25%, de son poids total. Ce latex peut en outre contenir un ou plusieurs additifs choisis notamment parmi les agents complexants, les agents de transfert ou les agents limiteurs de chaînes.

L'invention a aussi pour objet, une composition cosmétique, dermocosmétique, dermopharmaceutique ou pharmaceutique, caractérisée en ce qu'elle comprend comme composé épaississant, au moins un latex inverse tel que défini précédemment.

La composition cosmétique, dermocosmétique, dermopharmaceutique ou pharmaceutique définie ci-dessus, comprend généralement de 0,1 % à 10 % et plus particulièrement entre 0,5% et 5% en poids dudit latex inverse. Elle se présente notamment, sous la forme d'un lait, d'une lotion, d'un gel, d'une crème, d'un savon, d'un bain moussant, d'un baume, d'un shampooing ou d'un après shampooing.

De façon générale, ledit latex inverse, peut remplacer avantageusement les produits vendus sous le nom SEPIGEL^{™} 305 ou SEPIGEL^{™} 501 par la demanderesse, dans les compositions cosmétiques, dermopharmaceutiques ou pharmaceutiques, car il présente aussi une bonne compatibilité avec les autres excipients utilisés pour la préparation de formulations telles que les laits, les lotions, les crèmes, les savons, les bains moussants, les baumes, les shampooings ou les après shampooings. Il peut aussi être utilisé en combinaison avec lesdits SEPIGEL. Il est notamment compatible avec les concentrés décrits et revendiqués dans les publications internationales WO 92/06778, WO 95/04592, WO95/13863 ou FR 2734 496 ou avec les agents tensioactifs décrits dans WO 93/08204.

Il est particulièrement compatible avec le MONTANOV^{™} 68, le MONTANOV^{™} 82, le MONTANOV^{™} 202 le MONTANOV^{™} WO18, le MONTANOV^{™}S ou le SEPIPERL^{™} N. Il peut également être utilisé dans des émulsions du type de celles décrites et revendiquées dans EP 0 629 396 et dans les dispersions aqueuses cosmétiquement ou physiologiquement acceptables avec un composé organo-polysiloxane choisi, par exemple parmi ceux décrits dans WO 93/05762 ou dans WO 93/21316. Il peut également être utilisé pour former des gels aqueux à pH acide cosmétiquement ou physiologiquement acceptables, tels que ceux décrit dans WO 93/07856 ; il peut encore être utilisé en association avec des celluloses non-ioniques, pour former par exemple des gels de coiffage, tels que ceux décrits dans EP 0 684 024 ou encore en association avec des esters d'acides gras et de sucre, pour former des compositions pour le traitement du cheveux ou de la peau telles que celles décrites dans EP 0 603 019. ou encore dans les shampooings ou après shampooings tels que décrits et revendiqués dans WO 92/21316 ou enfin en association avec un homo polymère anionique tels que le CARBOPOL^{™} pour former des produits de traitement des cheveux comme ceux décrits dans DE 195 23596. Il est également compatible avec de nombreux principes actifs, tels que par exemple, les agents autobronzants comme le dihydroxyacétone (DHA) ou les agents anti-acné ; il peut donc être introduit dans des compositions auto-bronzantes comme celles revendiquées dans EP 0 715 845, EP 0604249, EP 0576188 ou dans WO 93/07902. Il est également compatible avec les dérivés N-acylés d'aminoacides, ce qui permet son utilisation dans des compositions apaisantes notamment pour peaux sensibles, telles que celles décrites ou revendiquées dans WO 92/21318, WO 94/27561 ou WO 98/09611. Il est aussi compatible avec les acides glycoliques, avec l'acide lactique, avec l'acide salicylique les rétinoïdes, le phénoxy éthanol, les sucres, le glycéraldéhyde, les xanthanes, les acides de fruit, et les divers polyols utilisés dans la fabrication de formulations cosmétiques.

L'invention a donc aussi pour objet, l'utilisation d'un latex inverse tel que défini précédemment, pour préparer une composition cosmétique, dermocosmétique, dermopharmaceutique ou pharmaceutique.

Les exemples qui suivent ont pour but d'illustrer la présente invention sans toutefois la limiter. Ils montrent que les nouveaux latex inverses n'irritent pas la peau et que leurs propriétés physiques permettent leur utilisation dans la préparation de compositions cosmétiques, dermopharmaceutiques ou pharmaceutiques plus particulièrement destinées au traitement des peaux sensibles.

### A) Exemple de préparation d'une composition selon l'invention

### Exemple 1 : Latex inverse d'un copolymère AMPS (sel de Na) / acide acrylique (sel de Na), réticulé au méthylène bis(acrylamide), dans un mélange de triglycéride d'acides gras comportant de 8 atomes de carbone (Composition 1)

a) - On charge dans un bécher sous agitation :
   - 55,86 g d'acide acrylique glacial,
   - 428,25 g d'une solution commerciale à 55% de 2-méthyl 2-[(1-oxo 2-propènyl) amino] 1-propanesulfonate de sodium,
   - 32,34 g d'une solution aqueuse à 48% en poids d'hydroxyde de sodium,
   - 0,46 g de diéthylènetriaminepentacétate de sodium,
   - 0,106 g de méthylène bis(acrylamide),
   - Le pH de cette solution aqueuse est ajusté à 4,9 et on rajoute de l'eau permutée de manière à porter la masse de la phase aqueuse à 625,61 g.
b) - On prépare une phase organique en mélangeant :
   - 57,1 g d'ISOPAR^{™} G,
   - 228,25 g de triglycéride d'acides gras en C₈ - C₁₀ commercialisé en France, par la société STEARINERIE DUBOIS & Fils, sous le nom Triglycérides C₈C₁₀ 5545.
   - 8,83 g d'HYPERMER^{™} B246 (copolymère octadécène / anhydride maléique commercialisé par la société CHEVRON Chemicals),
   - 1,47 g de résine PA18 et
   - 0,26 g d'azo bis(isobutyronitrile).
c) - La phase aqueuse est introduite progressivement dans la phase organique et l'ensemble est agité fortement au moyen d'un agitateur ULTRA-TURRAX^{™} commercialisé par IKA. L'émulsion obtenue est alors transférée dans un réacteur de polymérisation, soumise à un barbotage d'azote puis refroidit à environ 5-6°C. On ajoute alors 5 ml d'une solution contenant 0,384% en poids d'hydroperoxyde de cumène dans l'ISOPAR^{™} G, puis après homogénéisation de la solution, une solution aqueuse de métabisulfite de sodium (0,1% dans l'eau) pendant environ 60 minutes et en laissant monter la température jusqu'à la température de polymérisation. On maintient alors le milieu réactionnel pendant environ 90 minutes à cette température, à l'issu desquelles le mélange obtenu est chauffé sous vide partiel pour éliminer l'ISOPAR^{™} G et environ 10 kg d'eau. Le mélange obtenu est refroidi jusqu'à 35° environ. On introduit lentement 10,1 g d'huile de ricin éthoxylée à 40 moles d'oxyde d'éthylène (SIMULSOL^{™} OL50) et on obtient l'émulsion eau dans huile désirée.

### Evaluation des propriétés

Viscosité dans l'eau à 3% du latex (Brookfield RVT Mobile 6, vitesse 5) :
η = 106 000 mPa.s
Viscosité à 3% du latex dans de l'eau salée (NaCl 0,1%) (Brookfield RVT Mobile 3, vitesse 5) : η = 37 000 mPa.s.

### B Propriétés de la composition selon l'invention

### a) Influence du rayonnement UV sur la stabilité

On constate que les gels préparés avec la composition 1 est très stable au rayonnement UV; leur viscosité n'ayant pas varié après 14 jours d'exposition.

### b) Influence du pH sur la viscosité

La viscosité des gels préparés avec la composition 2 est très stable au pH dans l'intervalle pH = 4 à pH = 8

### c) Etude comparative de la tolérance

La tolérance locale épicutanée d'une série de gels-crèmes, contenant 3 % et 5 % en poids de la composition 1 préparée comme décrit ci-dessus, a été déterminée et comparée à celle observée avec un latex inverse d'un copolymère AMPS/acrylate de sodium réticulé au méthylène bis(acrylamide), dans l'isohexadécane (Composition A), selon le protocole suivant :
On applique la composition 1, à une surface d'environ 50 mm² de la région sous-scapulaire gauche, de la peau du dos de 38 volontaires sains, parmi lesquels 19 ont une peau de type "peau japonaise" (PJ) et 19 une peau de type "peau caucasienne" (PC). Le contact est maintenu pendant 48 heures sous timbre occlusif.

Cette application est aussi réalisée dans les mêmes conditions avec un timbre seul (sans composition) en tant que témoin négatif.

L'observation clinique de la surface de peau ainsi traitée est réalisée 30 minutes puis 24 heures après la dépose desdits timbres. Ces observations sont faites par comparaison à la surface non traitée témoin négatif.

La quantification de l'irritation cutanée, selon une échelle numérique allant de 0 à 4 (0 : pas d'effet ; 1 : effet très léger ; 2 : effet net ; 3 et 4: effet modéré à sévère selon les réactions), est réalisée pour chacune des réactions éventuellement observées à savoir : érythème, oedème, vésicules, sécheresse de la peau, rugosité de la peau et la réflectivité de la peau.

Les indices de tolérance cutanée (IC), consignés dans le tableau suivant, expriment la moyenne de la somme des effets quantifiés relevés sur chaque volontaire :
IC = 0 signifie qu'aucune irritation n'a été observée,
IC = 0,5 signifie que le produit est statistiquement bien toléré,
IC > 0,5 signifie que le produit engendre une intolérance. (Composition A).

| | Indice de tolérance cutanée de gels à 5% | |
|---|---|---|
| | PJ | PC |
| | | |
| composition 1 | 0,05 | 0,0 |
| composition A | 0,95 | 0,47 |

Ces résultats montrent que de façon inattendue, le triglycéride d'acides gras de la composition préparée à l'exemple 1, pontentialise la tolérance cutanée du polymère du latex inverse.

### C) Exemples de formulations préparées avec les compositions selon l'invention

### Exemple 2 : Emulsion satinée pour le corps

### FORMULE

| | | |
|---|---|---|
| A | SIMULSOL^{™} 165 : | 5,0% |
| | LANOL^{™} 1688 : | 8,50% |
| | Beurre de Karité : | 2% |
| | Huile de paraffine : | 6,5% |
| | LANOL^{™} 14M : | 3% |
| | LANOL^{™} S : | 0,6% |
| B | Eau : | 66,2% |
| C | MICROPEARL^{™} M 100 : | 5% |
| D | Composition 1 : | 3% |
| E | SEPICIDE^{™} CI : | 0,3% |
| | SEPICIDE^{™} HB : | 0,5% |
| | MONTEINE^{™} CA : | 1% |
| | Parfum : | 0,20% |
| | Acétate de vitamine E : | 0,20% |
| | Sodium pyrrolidinonecarboxylate : | 1% |

### MODE OPERATOIRE

Ajouter C dans B, émulsionner B dans A à 70°C, puis ajouter D à 60°C puis E à 30°C.

### Exemple 3 : Crème H/E

### FORMULE

| | | |
|---|---|---|
| A | SIMULSOL^{™} 165 : | 5,0% |
| | LANOL^{™} 1688 : | 20,0% |
| | LANOL^{™} P : | 1,0% |
| B | Eau : | q.s. 100% |
| C | Composition 1 : | 2,50% |
| D | SEPICIDE^{™} CI : | 0,20% |
| | SEPICIDE^{™} HB : | 0,30% |

### MODE OPERATOIRE

Introduire B dans A vers 75°C ; ajouter C vers 60°C, puis D vers 45°C.

### Exemple 4 : Gel solaire non gras

### FORMULE

| | | |
|---|---|---|
| A | Composition 1 : | 3,00% |
| | Eau : | 30% |
| B | SEPICIDE^{™} CI : | 0,20% |
| | SEPICIDE^{™} HB : | 0,30% |
| | Parfum : | 0,10% |
| C | Colorant : | q.s.p |
| | Eau: | 30% |
| D | MICROPEARL^{™} M 100 : | 3,00% |
| | Eau : | q.s.p 100% |
| E | Huile de silicone : | 2,0% |
| | PARSOL^{™} MCX : | 5,00% |

### MODE OPERATOIRE

Introduire B dans A; ajouter C, puis D, puis E.

### Exemple 5 : Gel de massage

### FORMULE

| | | |
|---|---|---|
| A | Composition 1 : | 3,5% |
| | Eau : | 20,0% |
| B | Colorant : | 2 gouttes/100g |
| | Eau : | q. s. |
| C | Alcool: | 10% |
| | Menthol : | 0,10% |
| D | Huile de silicone : | 5,0% |

### MODE OPERATOIRE

Ajouter B dans A, puis ajouter au mélange, C puis D

### Exemple 6 : Lait corporel

### FORMULE

| | |
|---|---|
| MONTANOV^{™} S : | 3,5% |
| LANOL^{™} 37T : | 8,0% |
| SOLAGUM^{™} L : | 0,05% |
| Eau : | q.s.p.100% |
| Benzophénone : | 2,0% |
| Diméthicone 350cPs : | 0,05% |
| Composition 1 : | 0,8% |
| Conservateur : | 0,2% |
| Parfum : | 0,4% |

### Exemple 7 : Crème hydratante pour peaux grasses

### FORMULE

| | |
|---|---|
| MONTANOV^{™} 68 : | 5% |
| Cétylstéaryloctanoate : | 8% |
| Octyl palmitate: | 2% |
| Eau : | q.s.p.100% |
| Composition 1 : | 0,6% |
| MICROPEARL^{™} M100 : | 3,0% |
| Mucopolysaccharides : | 5% |
| SEPICIDE^{™} HB : | 0,8% |
| Parfum : | 0,3% |

### Exemple 8 : Crème aux AHA pour peaux sensibles

### FORMULE

| | |
|---|---|
| Mélange de lauryl aminoacides : | 0,1 % à 5% |
| Aspartate de magnésium et de potassium : | 0,002% à 0,5% |
| LANOL^{™} 99 : | 2% |
| MONTANOV^{™} 68 : | 5,0% |
| Eau : | q.s.p.100% |
| Composition 1 : | 1,50% |
| Acide gluconique : | 1,50% |
| Tri éthylamine : | 0,9% |
| SEPICIDE™ HB : | 0,3% |
| SEPICIDE™ CI : | 0,2% |
| Parfum : | 0,4% |

### Exemple 9 : Lait démaquillant

### FORMULE

| | |
|---|---|
| SEPIPERL^{™} N : | 3% |
| PRIMOL^{™} 352 : | 8,0% |
| Huile d'amandes douces : | 2% |
| Eau : | q.s.p.100% |
| Composition 1 : | 0,8% |
| Conservateur : | 0,2% |

### Exemple 10 : Crème aux AHA

### FORMULE

| | | |
|---|---|---|
| A | MONTANOV™ 68 : | 5,0% |
| | LIPACIDE^{™} PVB: | 1,05% |
| | LANOL^{™} 99 : | 10,0% |
| B | eau: | q.s.p.100% |
| | Acide gluconique : | 1,5% |
| | TEA (triéthanolamine) : | 0,9% |
| C | Composition 1 : | 1,5% |
| D | Parfum: | 0,4% |
| | SEPICIDE^{™} HB: | 0,2% |
| | SEPICIDE^{™} CI: | 0,4% |

### Exemple 11 : Lait solaire au monoï de Tahiti

### FORMULE

| | | |
|---|---|---|
| A | Monoï de Tahiti: | 10% |
| | LIPACIDE^{™} PVB : | 0,5% |
| | Composition 2 : | 2,2% |
| B | Eau : | q.s.p. 100% |
| C | Parfum : | 0,1% |
| | SEPICIDE^{™} HB : | 0,3% |
| | SEPICIDE^{™} CI : | 0,1% |
| | PARSOL^{™} MCX : | 4,0% |

### Exemple 12 : Soin solaire pour le visage

### FORMULE

| | | |
|---|---|---|
| A | Cyclométhicone et diméthiconol : | 4,0% |
| | Composition 2 : | 3,5% |
| B | Eau : | q.s.p.100% |
| C | Parfum : | 0,1% |
| | SEPICIDE^{™} HB : | 0,3% |
| | SEPICIDE^{™} CI : | 0,21% |
| | PARSOL^{™} MCX : | 5,0% |
| | Micatitane : | 2,0% |
| | Acide lactique : | q.s.p. pH = 6,5 |

Les caractéristiques des produits utilisés dans les exemples précédents, sont les suivantes :
Le MONTANOV^{™} 68 (cétéaryl glucoside, alcool cétéarylique), est une composition auto-émulsionnable telle que celles décrites dans WO 92/06778, commercialisée par la société SEPPIC.
Le MICROPEARL^{™} M 100 est une poudre ultra fine au toucher très doux et à action matifiante commercialisée par la société MATSUMO.
Le SEPICIDE^{™} CI, imidazoline urée, est un agent conservateur commercialisé par la société SEPPIC.
Le SIMULSOL^{™} 165 est du stéarate de glycérol auto-émulsionnable commercialisée par la société SEPPIC.
Le LANOL^{™} 1688 est un ester émollient à effet non gras commercialisé par la société SEPPIC.
Le LANOL^{™} 14M et le LANOL^{™} S sont des facteurs de consistance commercialisés par la société SEPPIC.
Le SEPICIDE^{™} HB , qui est un mélange de phénoxyéthanol, de méthylparaben, d'éthylparaben, de propylparaben et de butylparaben, est un agent conservateur commercialisé par la société SEPPIC.
Le MONTEINE^{™} CA est un agent hydratant commercialisé par la société SEPPIC.
Le LANOL^{™} P est un additif à effet stabilisant commercialisé par la société SEPPIC.
Le SEPIPERL^{™} N est un agent nacrant, commercialisé par la société SEPPIC, à base d'un mélange d'alkyl polyglucosides tels que ceux décrits dans WO 95/13863.
Le MONTANOV^{™} S est un agent nacrant, commercialisé par la société SEPPIC, à base d'un mélange d'alkyl polyglucosides tels que ceux décrits dans WO 95/13863.
Le LANOL^{™} 99 est de l'isononyl isononanoate commercialisé par la société SEPPIC.
Le LANOL^{™} 37T est du triheptanoate de glycérol, commercialisé par la société SEPPIC.
Le SOLAGUM^{™} L est un carraghénane commercialisé par la société SEPPIC.
Le PARSOL^{™} MCX est du paraméthoxycinnamate d'(éthyl hexyle) commercialisé par la société GIVAUDAN.
Le LIPACIDE^{™} PVB, est un hydrolysat de protéines de blé palmitoylé, commercialisée par la société SEPPIC.

## Revendications

1. Composition comprenant une phase huile, une phase aqueuse, au moins un agent émulsifiant de type eau dans huile (E/H), au moins un agent émulsifiant de type huile dans eau (H/E), sous forme d'un latex inverse auto-inversible comprenant de 20% à 70% en poids, et de préférence de 25% à 50% en poids, d'un polyélectrolyte branché ou réticulé, choisi parmi :
- un homopolymère de l'acide 2-méthyl 2-[(1-oxo 2-propènyl) amino] 1-propanesulfonique partiellement ou totalement salifiée sous forme d'un sel de métal alcalin, de sel d'ammonium, de sel de monoéthanolamine ou de sel de lysine ;
- un homopolymère de l'acide acrylique, de l'acide méthacrylique, de l'acide itaconique ou de l'acide maléique partiellement ou totalement salifié ;
- un copolymère de l'acide 2-méthyl 2-[(1-oxo 2-propènyl) amino] 1-propanesulfonique partiellement ou totalement salifiée sous forme d'un sel de métal alcalin, de sel d'ammonium, de sel de monoéthanolamine ou de sel de lysine, copolymérisé ou bien avec de l'acide acrylique, de l'acide méthacrylique, de l'acide itaconique ou de l'acide maléique partiellement ou totalement salifié , ou bien avec au moins un monomère neutre choisi parmi l'acrylate de (2-hydroxy éthyle), l'acrylate de (2,3-dihydroxy propyle), le méthacrylate de (2-hydroxy éthyle), le méthacrylate de (2,3-dihydroxy propyle), ou un dérivé éthoxylé de poids moléculaire compris entre 400 et 1000, de chacun de ces esters ;
- un copolymère de l'acide acrylique, de l'acide méthacrylique, de l'acide itaconique ou de l'acide maléique partiellement ou totalement salifié, copolymérisé avec au moins un monomère neutre choisi parmi l'acrylate de (2-hydroxy éthyle), l'acrylate de (2,3-dihydroxy propyle), le méthacrylate de (2-hydroxy éthyle), le méthacrylate de (2,3-dihydroxy propyle), ou un dérivé éthoxylé de poids moléculaire compris entre 400 et 1000, de chacun de ces esters ;
**caractérisé en ce que** la phase huile est essentiellement constituée d'un composé de formule (I) :
R₁-(C=O)-O-[[CH₂-CH[O-[C(=O)]ₘ-R₂]-CH₂-O]ₙ-[C(=O)]ₚ]_{q}-R₃ (I)
dans laquelle :
R₁ représente une chaîne hydrocarbonée saturée ou insaturée, linéaire ou ramifiée comportant de 7 à 30 atomes de carbone,
R₂ représente indépendamment de R₁, un atome d'hydrogène, une chaîne hydrocarbonée saturée ou insaturée linéaire ou ramifiée comportant de 7 à 30 atomes de carbone,
R₃ représente indépendamment de R₁ ou de R₂, un atome d'hydrogène ou une chaîne hydrocarbonée saturée ou insaturée, linéaire ou ramifiée comportant de 1 à 30 atomes de carbone,
m, n et p sont indépendamment l'un de l'autre, égaux à 0 ou à 1, et q est égal à 1.

2. Composition telle que définie à la revendication 1, pour laquelle dans la formule (I), R₁, R₂ et R₃ représentent, indépendamment les uns des autres, un radical choisi parmi les radicaux heptyle, octyle, nonyle, décyle, undécyle, dodécyle, tridécyle, tétradécyle, pentadécyle, hexadécyle, heptadécyle, octadécyle, nonadécyle, eicosyle, heneicosyle, docosyle, heptadécènyle, eicosènyle, heneicosènyle, docosènyle, heptadécadiènyle ou décènyle.

3. Composition telle que définie à la revendication 2, pour laquelle dans la formule (I), le groupe R₁-C(=O)- représente l'un des radicaux octanoyle, décanoyle, undécylènoyle, dodécanoyle, tétradécanoyle, hexadécanoyle, octadécanoyle, eicosanoyle, docosanoyle, 8-octadécènoyle, éicosènoyle, 13-docosènoyle, 9,12-octadécadiènoyle ou 9,12,15-octadécatriénoyle.

4. Composition telle que définie à l'une quelconque des revendications 1 à 3, dans laquelle la phase huile est essentiellement constituée d'un composé de formule (Ia) :
R₁-(C=O)-O-CH₂-CH[O-[C(=O)]ₘ-R₂]-CH₂-O-[C(=O)]ₚ-R₃ (Ia)
correspondant à la formule (I), dans laquelle q et n sont égaux à 1, ou d'un mélange de composés de formule (Ia).

5. Composition telle que définie à la revendication 4 dans laquelle la phase huile est essentiellement constituée d'un composé de formule (Ia₁) :
R₁-(C=O)-O-CH₂-CH(OH)-CH₂-OH (Ia₁)
correspondant à la formule (Ia) dans laquelle m et p sont égaux à 0 et R₂ et R₃ représentent un atome d'hydrogène.

6. Composition telle que définie à la revendication 4 dans laquelle la phase huile est essentiellement constituée d'un composé de formule (Ia₂) :
R₁-(C=O)-O-CH₂-CH(OH)-CH₂-O-C(=O)-R₃ (Ia₂)
correspondant à la formule (Ia) dans laquelle p est égal à 1, m est égal à 0 et R₂ représente un atome d'hydrogène.

7. Composition telle que définie à la revendication 4 dans laquelle la phase huile est essentiellement constituée d'un composé de formule (Ia₃) :
R₁-(C=O)-O-CH₂-CH[O-C(=O)-R₂]-CH₂-O-C(=O)-R₃ (Ia₃)
correspondant à la formule (Ia) dans laquelle m et p sont égaux à 1.

8. Composition telle que définie aux revendications 5 à 7, dans laquelle la phase huile, est un mélange de composés de formules (Ia₁), (Ia₂) et/ou (Ia₃).

9. Composition telle que définie à l'une quelconques des revendications 1 à 8, dans laquelle le ou les agents émulsifiants du type eau dans huile, sont choisis parmi le monooléate de sorbitan, l'isostéarate de sorbitan ou l'oléate de sorbitan éthoxylé avec 5 moles d'oxyde d'éthylène.

10. Composition telle que définie à l'une quelconques des revendications 1 à 9, dans laquelle le ou les agents émulsifiants du type huile dans eau sont choisis parmi l'oléate de sorbitan éthoxylé avec 20 moles d'oxyde d'éthylène, l'huile de ricin éthoxylée à 40 moles d'oxyde d'éthylène, le laurate de sorbitan éthoxylé à 20 moles d'oxyde d'éthylène, l'alcool laurique éthoxylé à 7 moles d'oxyde d'éthylène.

11. Composition telle que définie à l'une des revendications 1 à 10, dans laquelle le polyélectrolyte est un homopolymère d'acide acrylique partiellement ou totalement salifié sous forme de sel de sodium ou de sel d'ammonium.

12. Composition telle que définie à l'une des revendications 1 à 10, dans laquelle le polyélectrolyte est un copolymère d'acide 2-méthyl 2-[(1-oxo 2-propènyl) amino] 1-propanesulfonique partiellement ou totalement salifié (a), et d'acrylate de (2-hydroxy éthyle) (b), dans un rapport molaire (a) / (b) compris entre 30 / 70 et 90 / 10 et tout particulièrement 50 / 50 et 90 / 10.

13. Composition telle que définie à l'une des revendications 1 à 10, dans laquelle le polyélectrolyte est un copolymère de sel de sodium de sel d'ammonium, de sel de monoéthanolamine ou de sel de lysine de l'acide 2-méthyl 2-[(1-oxo 2-propènyl) amino] 1-propanesulfonique (a₁) et d'acide acrylique partiellement ou totalement salifié sous forme de sel de sodium, de sel d'ammonium, de sel de monoéthanolamine ou de sel de lysine (c₁), dans un rapport molaire (a₁) / (c₁), compris entre 30 / 70 et 90/10 et tout particulièrement entre 30 / 70 et 45/55.

14. Composition telle que définie à l'une des revendications 1 à 10, dans laquelle le polyélectrolyte est un copolymère de sel de sodium ou de sel d'ammonium de l'acide 2-méthyl 2-[(1-oxo 2-propènyl) amino] 1-propanesulfonique (a₂) et d'acrylamide (d), dans un rapport molaire (a₂) / (d), compris entre 50 / 50 et 30/70.

15. Composition telle que définie à l'une quelconque des revendications 1 à 14, **caractérisée en ce que** le polyélectrolyte est réticulé et/ou branché avec un composé diéthylénique ou polyéthylénique dans la proportion molaire exprimée par rapport aux monomères mis en oeuvre, de 0,005% à 1%, et de préférence de 0,01 % à 0,5 % et, plus particulièrement de 0,1 % à 0,25 %.

16. Composition cosmétique, dermocosmétique, dermopharmaceutique ou pharmaceutique, **caractérisée en ce qu'**elle comprend de 0,1 % à 10 % en poids, de la composition telle que définie à l'une quelconque des revendications 1 à 15.
